# FASCICULE DE BREVET EUROPEEN

(11) **EP 3 391 912 B1**
(45) Date de publication et mention de la délivrance du brevet: **14.07.2021**
(21) Numéro de dépôt: 18305489.9
(22) Date de dépôt: 20.04.2018
(51) Int. Cl.: A61L 2/22, A61L 2/18, A61L 2/26

(54) **INSTALLATION POUR LE TRAITEMENT DE DÉSINFECTION DE CHARIOTS HOSPITALIERS ET SON PROCÉDÉ DE MISE EN OEUVRE**
ANLAGE ZUR DESINFEKTIONSBEHANDLUNG VON KRANKENHAUSWAGEN UND IHRE ANWENDUNG
FACILITY FOR DISINFECTION TREATMENT OF HOSPITAL CARTS AND METHOD FOR IMPLEMENTING SAME

(30) Priorité: 20.04.2017 FR 1753421
(43) Date de publication de la demande: 24.10.2018
(73) Titulaire: Techni Brume Diffusion SARL - TBD, 49280 La Séguinière (FR)
(72) Inventeur: CHAVANTRE, Didier, 44300 Nantes (FR); MONTOURCY, Thierry, 03400 Yzeure (FR)
(74) Mandataire: Jacobacci Coralis Harle

(56) Documents cités:
- WO-A1-2004/002541
- WO-A2-2010/127296
- DE-A1- 2 136 561
- US-A1- 2008 006 309

## Description

### DOMAINE TECHNIQUE AUQUEL SE RAPPORTE L'INVENTION

La présente invention concerne de manière générale le domaine des installations de désinfection.
Elle concerne plus particulièrement une installation pour le traitement de désinfection de chariots hospitaliers, en particulier des chariots de transport de repas ou de linge.

Par la notion de « chariot hospitalier », on entend tout chariot pour le transport de repas (avec ou sans plateaux), de linge ou similaires, dans les établissements de santé type hôpitaux, cliniques ou équivalents.

### ARRIERE-PLAN TECHNOLOGIQUE

Du fait de leur utilisation en milieu médicalisé et de leur vocation à être déplacés dans différents espaces susceptibles d'être contaminés, les chariots de transport de repas ou de linge nécessitent d'être désinfectés de manière régulière, pour limiter les risques sanitaires.

Pour cela il existe des structures de caisson de désinfection munis d'au moins une porte pour l'introduction et la sortie des chariots à traiter, et qui intègrent des moyens de désinfection constitués d'une rampe d'aspersion d'un produit liquide contenant un agent de traitement hydrosoluble adapté à la désinfection des chariots.

La rampe d'aspersion consiste en une conduite munie d'une entrée dans le caisson et d'une sortie dudit caisson. Le produit liquide de traitement est propulsé au travers de buses d'aspersion qui équipent la rampe au moyen d'une pompe dont le fonctionnement est géré par un automate programmable.

Une installation de désinfection présentée dans Le document WO-2010/12 7296 décrit un appareil de décontamination comprenant une enceinte intégrant un premier système de décontamination et un second système de décontamination.

L'un au moins de ces deux systèmes de décontamination peut consister en un produit liquide chimique délivré sous la forme d'un brouillard.

Des moyens de renouvellement d'air sont également prévus pour l'extraction de l'air vicié et l'apport d'air neuf au sein de l'appareil.

Le document WO-2004/002541 décrit de son côté un sas, placé entre un volume extérieur et une enceinte aseptique de qualité pharmaceutique, adapté pour la désinfection d'une combinaison équipant un opérateur.

Le sas est équipé de ventilateurs, de tuyères pour la projection d'un liquide désinfectant sur la combinaison, et de moyens de séchage de la combinaison par ventilateurs.

Le document DE-2136 561 décrit un dispositif placé dans un local pour désinfecter des meubles dans les hôpitaux, en particulier des lits.

Ce dispositif comprend des moyens pour supporter/déplacer le lit et un système de rampe mobile équipée de buses de projection de liquide désinfectant. Ces buses de projection sont reliées à un réservoir pressurisé de liquide désinfectant.

Il est également prévu des moyens d'extraction de l'air interne vicié du local, et des moyens d'entrée d'air neuf.

Le document US-2008/006309 décrit une installation de désinfection de chariots de supermarché, comprenant un caisson muni d'une porte d'entrée et d'une porte de sortie, et au sein duquel sont prévus des moyens de cheminement des chariots pour leur faire subir trois étapes successives de traitement entre l'entrée et la sortie.

Dans la 1ère étape, un premier groupe de moyens de traitement est adapté pour laver les chariots avec de l'eau ou de l'eau associée à un détergeant.

Dans la 2ème étape, un second groupe de moyens de traitement, successif au premier, est adapté pour désinfecter les chariots avec un produit liquide désinfectant.

Et au cours de la 3ème étape, un troisième groupe de moyens de traitement, successif au second, est adapté pour rincer et sécher les chariots.

Les produits liquides de traitement peuvent être délivrés par des buses sous la forme d'un brouillard.

Cependant les installations actuelles posent des problèmes d'efficacité et elles sont en outre relativement onéreuses. Ces installations sont par ailleurs consommatrices en eau. La projection liquide expose également les organes sensibles (connections, isolations...), et rend les appareils plus vulnérables.

### OBJET DE L'INVENTION

Afin de remédier aux inconvénients précités de l'état de la technique, la présente invention propose une installation pour le traitement de désinfection de chariots hospitaliers, en particulier des chariots de transport de repas ou de linge laquelle installation est du type comprenant :
- un caisson délimitant un volume de traitement adapté pour la réception d'une pluralité desdits chariots et muni d'au moins une porte pour permettre l'entrée et la sortie desdits chariots, et
- des moyens de désinfection comprenant au moins une conduite interne munie d'une entrée dans ledit caisson et d'une sortie dudit caisson, laquelle conduite interne est munie de moyens de diffusion, disposés à l'intérieur dudit caisson, pour diffuser dans ledit volume de traitement un produit liquide de traitement contenant un agent de traitement hydrosoluble adapté au traitement de désinfection desdits chariots,
lesquels
moyens de diffusion comprennent au moins une buse de brumisation, lesquels moyens de désinfection comprennent
des moyens permettant d'alimenter ladite entrée de conduite interne avec ledit produit liquide de traitement,
laquelle installation comprend encore une canalisation adaptée pour alimenter en air ledit volume de traitement et une canalisation équipée de moyens d'extraction adaptés pour extraire l'air vicié dudit volume de traitement,
et lequel caisson comprend au moins une ouverture d'entrée, munie de moyens d'obturation amovibles, associée à ladite canalisation adaptée pour alimenter en air ledit volume de traitement, et au moins une ouverture de sortie, munie de moyens d'obturation amovibles, associée à ladite canalisation équipée desdits moyens d'extraction adaptés pour extraire l'air vicié dudit volume de traitement,
caractérisé en ce que lesdits moyens de désinfection comprennent encore :
- des moyens permettant d'alimenter ladite entrée de conduite interne avec de l'air et
- des moyens permettant d'alimenter ladite entrée de conduite interne avec de l'eau.

D'autres caractéristiques non limitatives et avantageuses de l'installation de désinfection conforme à l'invention, prises individuellement ou selon toutes les combinaisons techniquement possibles, sont les suivantes :
- le caisson comporte un module d'entrée muni d'une porte d'entrée, et un module de sortie muni d'une porte de sortie, entre lesquels sont ménagés une pluralité de modules intermédiaires identiques, dont le nombre est fonction du volume de traitement recherché dudit caisson, ledit module d'entrée et ledit module de sortie étant assemblés de manière étanche avec ledit module intermédiaire adjacent, et lesdits modules intermédiaires étant assemblés entre eux de manière étanche ;
- l'installation comprend au moins une buse de brumisation ménagée sur ladite conduite interne au niveau dudit module d'entrée, au dessus du niveau de ladite porte d'entrée, et au moins une buse de brumisation ménagée sur ladite conduite interne au niveau dudit module de sortie, au dessus du niveau de ladite porte de sortie ;
- l'installation comprend des moyens de commande permettant de commander :
   - l'alimentation de ladite entrée de conduite interne, soit par le produit liquide de traitement, soit par de l'eau, soit par de l'air, et/ou
   - l'ouverture desdits moyens d'obturation amovibles des ouvertures d'entrée et de sortie du caisson ainsi que la mise en marche desdits moyens d'extraction, pour extraire l'air vicié dudit volume de traitement ;
- l'installation comporte un circuit fluidique comprenant :
   - un réservoir de produit liquide de traitement situé à l'extérieur dudit caisson,
   - une conduite principale d'alimentation s'étendant depuis ledit réservoir de produit liquide de traitement jusqu'à ladite entrée de conduite interne,
   - une conduite retour s'étendant depuis ladite sortie de conduite interne jusqu'audit réservoir de produit liquide de traitement,
   - une conduite d'alimentation en air s'étendant depuis une arrivée d'air jusqu'à ladite conduite principale d'alimentation,
   - une conduite d'alimentation en eau s'étendant depuis une arrivée d'eau jusqu'à ladite conduite principale d'alimentation, et
   - une conduite de sortie s'étendant depuis ladite sortie de conduite interne jusqu'à une sortie d'évacuation ;
   en outre différents clapets anti-retour sont agencés sur ledit circuit fluidique pour assurer le transport du produit liquide de traitement, de l'eau et de l'air, uniquement dans une direction en amont et en aval de ladite conduite interne ;
   et différentes vannes sont agencées sur ledit circuit fluidique, dont la position d'ouverture/fermeture est gérée par lesdits moyens de commande, adaptées pour autoriser :
   - l'alimentation de ladite entrée de conduite interne, soit par le produit liquide de traitement, soit par de l'eau, soit par de l'air,
   - l'évacuation de l'eau vers ladite sortie d'évacuation,
   - le retour dudit produit liquide de traitement et de l'air dans ledit réservoir ;
- l'installation comporte une pompe d'alimentation placée sur ladite conduite principale d'alimentation pour assurer l'alimentation de ladite entrée de conduite interne par le produit liquide de traitement,
   et ladite conduite d'alimentation en air, ainsi que ladite conduite d'alimentation en eau, viennent se raccorder à ladite conduite principale d'alimentation entre ladite pompe d'alimentation et ladite entrée de conduite interne ;
- l'installation comporte une pompe de gavage placée sur ladite conduite principale d'alimentation entre ledit réservoir de produit liquide de traitement et ladite pompe d'alimentation.
- l'installation comporte des moyens de détection de la présence de liquide dans ladite conduite retour, lesdits moyens de commande étant adaptés pour commander la diffusion du produit liquide de traitement par la ou les buses de brumisation uniquement en cas de détection de liquide dans ladite conduite retour.

L'invention propose également un procédé de désinfection de chariots hospitaliers au moyen d'une installation telle que définie ci-dessus, comprenant les étapes consistant à :
a/ introduire les chariots hospitaliers dans le volume de traitement du caisson,
b/ fermer ledit caisson de manière étanche,
c/ alimenter ladite conduite interne par ledit produit liquide de traitement, de manière adaptée pour diffuser ledit produit liquide de traitement dans ledit volume de traitement par la ou les buses de brumisation et former ainsi un brouillard de traitement,
d/ conserver le brouillard de traitement en contact avec lesdits chariots hospitaliers pendant un temps de contact adapté pour assurer leur désinfection,
e/ alimenter ladite conduite interne par de l'air, de manière adaptée pour enlever ledit produit liquide de traitement de ladite conduite interne et éventuellement desdites buses de brumisation,
f/ alimenter ladite conduite interne par de l'eau, de manière adaptée pour assurer son rinçage, et éventuellement le rinçage desdites buses de brumisation, et
   simultanément aux étapes e/ et/ou f/, ou après l'étape f/ :
g/ commander l'ouverture desdits moyens d'obturation amovibles des ouvertures d'entrée et de sortie du caisson et la mise en marche desdits moyens d'extraction, pour extraire l'air vicié dudit volume de traitement, et
h/ sortir lesdits chariots hospitaliers dudit caisson.

Ce procédé comprend encore de préférence une étape de remplissage complet de la conduite interne par ledit produit liquide de traitement, avant d'assurer la diffusion dudit produit liquide de traitement par lesdites buses de brumisation.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique de côté d'une installation de désinfection selon l'invention ;
- la figure 2 est une vue schématique de dessus de l'installation de désinfection de la figure 1 ;
- la figure 3 est une vue schématique de face de l'installation de désinfection illustrée sur les figures 1 et 2 ;
- la figure 4 est une vue schématique de côté de l'installation de désinfection des figures 1 à 3, illustrant le positionnement des chariots hospitaliers dans le volume de traitement du caisson ;
- la figure 5 est une vue schématique détaillant le circuit fluidique de l'installation de désinfection des figures 1 à 4.

### Installation

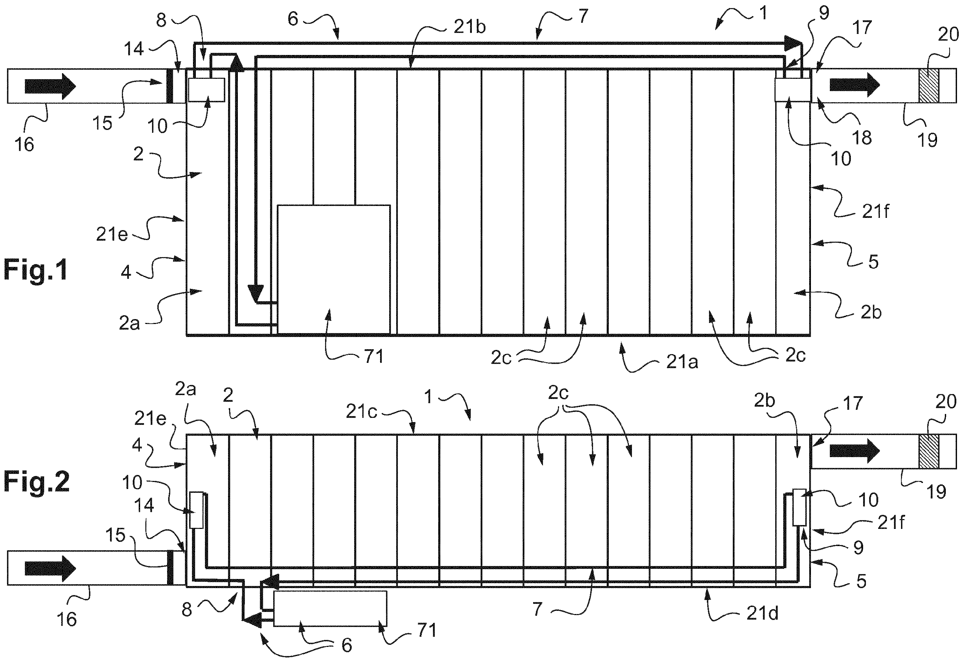

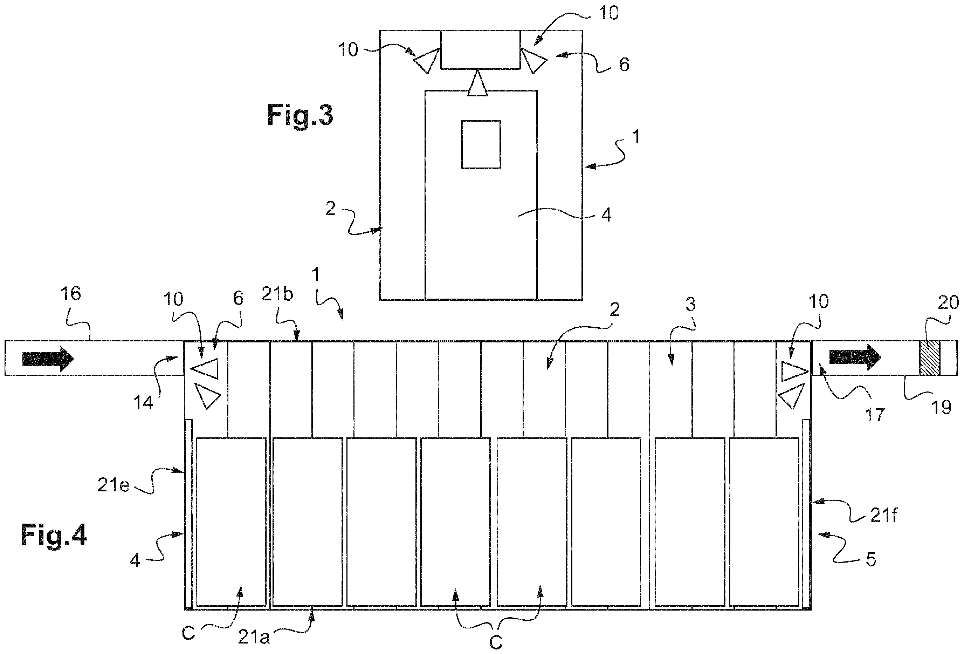

L'installation 1 représentée sur les figures 1 à 5 est adaptée pour le traitement de désinfection de chariots hospitaliers C, en particulier des chariots C dédiés au transport de repas ou de linge.
A titre purement indicatif de tels chariots peuvent avoir des dimensions de l'ordre de 0,80 m de largeur par 0,80 m de longueur, avec une hauteur de 1,30 m.

Cette installation de désinfection 1 comprend - un caisson 2 délimitant un volume de traitement 3 adapté pour la réception d'une pluralité desdits chariots C, muni d'une porte d'entrée 4 et d'une porte de sortie 5, et - des moyens de désinfection 6 comprenant au moins une conduite interne 7 munie d'une entrée 8 dans ledit caisson 2 et d'une sortie 9 dudit caisson 2, laquelle conduite interne 7 est munie de moyens de diffusion, comprenant au moins une buse de brumisation 10, disposés à l'intérieur dudit caisson 2, pour diffuser dans ledit volume de traitement 3 un produit liquide de traitement contenant un agent de traitement hydrosoluble adapté au traitement de désinfection desdits chariots C (par exemple du peroxyde d'hydrogène (H₂O₂)).

Sur les figures 1 et 2 la conduite interne 7 a été illustrée schématiquement à l'extérieur du caisson 2 par mesure de simplification de représentation.

Selon l'invention, les moyens de désinfection 6 comprennent encore :
- des moyens 11 permettant d'alimenter l'entrée 8 de la conduite interne 7 avec ledit produit liquide de traitement,
- des moyens 12 permettant d'alimenter ladite entrée 8 de conduite interne 7 avec de l'eau et
- des moyens 13 permettant d'alimenter ladite entrée 8 de conduite interne 7 avec de l'air ;
en outre, le caisson 2 comprend également - au moins une ouverture d'entrée 14, munie de moyens d'obturation amovibles 15, associée à une canalisation 16 adaptée pour alimenter en air ledit volume de traitement 3, et - au moins une ouverture de sortie 17, munie de moyens d'obturation amovibles 18, associée à une canalisation 19 équipée de moyens d'extraction 20 adaptés pour extraire l'air vicié dudit volume de traitement 3 (en assurant l'apport d'air neuf par la canalisation 16).

Le caisson 2 se présente sous la forme d'une enceinte de forme générale parallélépipédique comprenant un élément de plancher 21a, un élément de plafond 21b, deux parois latérales 21c et 21d, une paroi avant 21e munie de la porte d'entrée 4, et une paroi arrière 21f munie de la porte de sortie 5.

Ce caisson 2 est avantageusement conçu modulaire pour permettre d'adapter ses dimensions selon la quantité de chariots C que l'on souhaite traiter par cycle de traitement.

Pour cela, le caisson 2 comporte un module d'entrée 2a et un module de sortie 2b, entre lesquels sont ménagés une pluralité de modules intermédiaires 2c identiques ou similaires.
Le module d'entrée 2a comprend la paroi avant 21e et une partie de l'élément de plancher 21a, de l'élément de plafond 21b et des parois latérales 21c et 21d.
Le module de sortie 2b comprend la paroi arrière 21f et une partie de l'élément de plancher 21a, de l'élément de plafond 21b et des parois latérales 21c et 21d.
Les modules intermédiaires 2c se présentent sous une forme générale annulaire et comprennent une partie de l'élément de plancher 21a, une partie de l'élément de plafond 21b et une partie des parois latérales 21c et 21d.

Le module d'entrée 2a et le module de sortie 2b sont assemblés de manière étanche avec leur module intermédiaire 2c adjacent ; et les modules intermédiaires 2c sont assemblés entre eux également de manière étanche.
Les assemblages correspondants peuvent par exemple être réalisés au moyen de rivets avec interposition d'un joint d'étanchéité entre les couples de modules.

A titre purement indicatif, les parois avant 21e et arrière 21f des modules d'entrée 2a et de sortie 2b peuvent avoir une largeur de l'ordre de 1,90 m, et une hauteur de l'ordre de 2,50 m ; leur profondeur peut être de l'ordre de 0,35 m.
De leur côté, les modules intermédiaires 2c peuvent avoir une profondeur de l'ordre de 0,55 m.

Le nombre de modules intermédiaires 2c est fonction de la longueur souhaitée de l'installation de désinfection et donc du volume de traitement 3 recherché.
Par exemple 6 à 12 modules intermédiaires 2c peuvent être assemblés avec un module d'entrée 2a et un module de sortie 2b.

Le module d'entrée 2a comporte l'ouverture d'entrée 14 (munie des moyens d'obturation amovibles 15) à laquelle est raccordée la canalisation 16 d'alimentation en air ; et le module de sortie 2b comporte l'ouverture de sortie 17 associée à la canalisation 19 équipée des moyens d'extraction 20.

Les moyens d'obturation amovibles 15 et 18 de l'ouverture d'entrée 14 et de l'ouverture de sortie 17 consistent par exemple en un ou plusieurs volets pivotants.

Les moyens d'extraction 20 consistent en un extracteur d'air classique dimensionné en fonction du débit d'extraction recherché.

Bien entendu, les dimensions de la porte d'entrée 4 et celles de la porte de sortie 5 sont adaptées pour permettre l'entrée des chariots C dans le volume de traitement 3 du caisson 2, et leur sortie.

La porte d'entrée 4 et la porte de sortie 5 sont équipées de gâches électriques dont les manœuvres d'activation/désactivation sont gérées par les moyens de commande de l'installation de désinfection. Un système complémentaire d'ouverture manuelle de sécurité est prévu, par exemple en forme de câble permettant l'ouverture des portes de l'extérieur et en forme de loquet permettant l'ouverture des portes de l'intérieur.

Pour assurer une diffusion optimale du produit liquide de traitement au sein du volume de traitement 3, la ou les buses de brumisation 10 sont placées sur la conduite interne 7 au dessus du niveau des portes d'entrée 4 et de sortie 5. De la sorte la diffusion du produit de traitement est réalisée au dessus des chariots C à traiter.

De préférence, comme on peut le voir sur les figures 1 à 4, au moins une buse de brumisation 10 est ménagée sur conduite interne 7 au niveau du module d'entrée 2a, au dessus du niveau de la porte d'entrée 4, et au moins une buse de brumisation 10 est ménagée sur la conduite interne 7 au niveau du module de sortie 2b, au dessus du niveau de la porte de sortie 5.
En l'occurrence trois buses de brumisation 10 sont placées au dessus de la porte d'entrée 4 et trois buses de brumisation 10 sont placées au dessus de la porte de sortie 5.
On peut par exemple utiliser des buses de brumisation modèle TBD010 de la société TBD, 49280 La Séguinère, France (conformes aux buses de brumisation décrites dans le document FR-2 902 351).

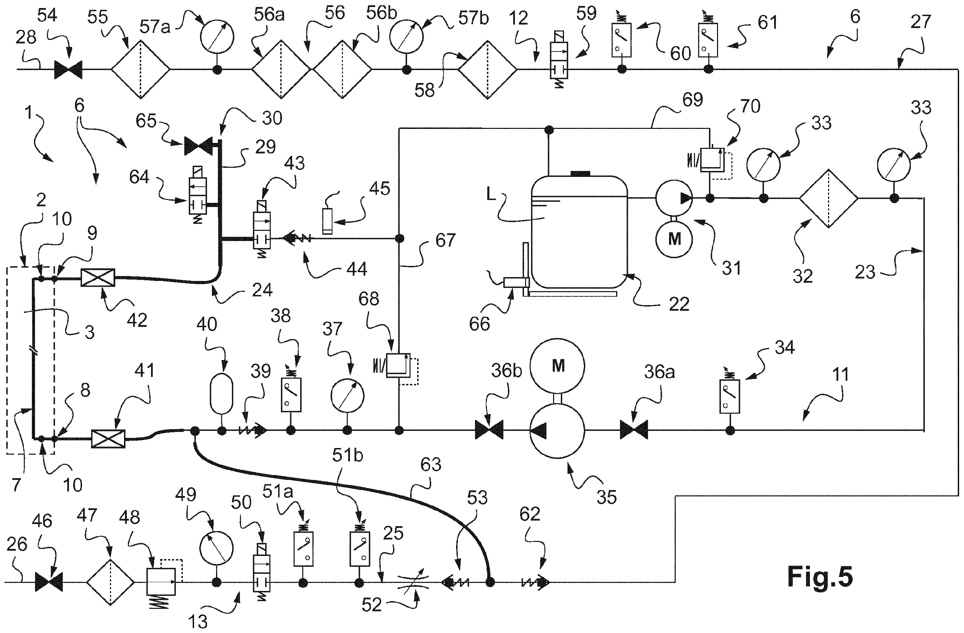

Comme précisé ci-dessus, les moyens de désinfection 6 comprennent un circuit fluidique 11, 12, 13, détaillé sur la figure 5, qui permet d'alimenter en produit liquide de traitement, en eau ou en air, l'entrée 8 de la conduite interne 7.

Comme on peut le voir sur cette figure 5, le circuit fluidique correspondant, situé à l'extérieur du caisson 2, comprend :
- un réservoir 22 de liquide de traitement L,
- une conduite principale d'alimentation 23 s'étendant depuis le réservoir 22 de liquide de traitement L jusqu'à l'entrée 8 de la conduite interne 7,
- une conduite retour 24 s'étendant depuis la sortie 9 de la conduite interne 7 jusqu'audit réservoir 22 de liquide de traitement L,
- une conduite 25 d'alimentation en air, s'étendant depuis une arrivée d'air 26 jusqu'à ladite conduite principale d'alimentation 23,
- une conduite 27 d'alimentation en eau s'étendant depuis une arrivée d'eau 28 jusqu'à ladite conduite principale d'alimentation 23, et
- une conduite de sortie 29 s'étendant depuis ladite sortie 9 de la conduite interne 7 (ou plus précisément ici depuis la conduite retour 24) jusqu'à une sortie d'évacuation 30.

La conduite principale d'alimentation 23 comprend, depuis le réservoir 22 de liquide de traitement L jusqu'à l'entrée 8 de la conduite interne 7, dans le sens amont/aval du fluide transporté : une pompe de gavage 31 (par exemple du type pompe à membrane avec actionneur électrique), un filtre 32 pour retenir les impuretés (par exemple un filtre 10 microns absolus) encadré par deux manomètres 33, un transmetteur de pression 34 (pour s'assurer du bon fonctionnement de l'installation), une pompe haute pression 35 adaptée pour assurer la diffusion du liquide de traitement L par les buses de brumisation 10 (par exemple une pompe tout inox sans joint sans huile pour eaux techniques), encadrée par deux vannes manuelles d'isolement 36a et 36b, un manomètre 37, un transmetteur de pression 38 (pour s'assurer de la bonne mise en pression du liquide afin de le transformer en brouillard), un clapet anti-retour taré 39 agencé pour empêcher le fluide transporté de s'orienter vers la pompe haute pression 35, un accumulateur 40 et un raccord mâle/femelle 41 qui permet de connecter hydrauliquement la conduite 23 à l'entrée 8 de la conduite interne 7.

La conduite retour 24 comprend, depuis la sortie 9 de la conduite interne 7 jusqu'au réservoir 22 de liquide de traitement L, dans le sens amont/aval du fluide transporté : un raccord mâle/femelle 42 qui permet de connecter hydrauliquement la canalisation 24 à ladite sortie 9 de la conduite interne 7, une électrovanne de commande retour 43, un clapet anti-retour taré 44 agencé pour empêcher le fluide transporté de s'orienter vers la conduite interne 7, et un capteur capacitif 45 permettant de détecter la présence d'un liquide dans ladite conduite retour 24.

De son côté, la conduite 25 d'alimentation en air s'étend depuis l'arrivée d'air 26 jusqu'à la conduite principale d'alimentation 23, qu'elle rejoint entre l'accumulateur 40 et le raccord mâle/femelle 41, et elle comprend, dans le sens amont/aval du fluide transporté : une vanne d'isolement 46, un filtre 47, un régulateur de pression 48, un manomètre 49, une électrovanne de commande d'air 50, un pressostat 51a (dont la fonction est de contrôler la bonne pression de l'air comprimé), un débistat 51b (dont la fonction est de vérifier l'étanchéité du circuit d'air et de s'assurer de la bonne alimentation de l'air comprimé), un limiteur de débit 52 et un clapet anti-retour taré 53 agencé pour empêcher le fluide transporté de s'orienter vers l'arrivée d'air 26.

La conduite 27 d'alimentation en eau comprend, depuis l'arrivée d'eau 28 jusqu'à la conduite principale d'alimentation 23, dans le sens amont/aval du fluide transporté : une vanne d'isolement 54, un premier système de filtre 55 pour retenir les impuretés grossières (par exemple un filtre 600 microns), un second système de filtre 56 pour retenir les impuretés plus fines (par exemple un filtre 25 microns 56a associé à un filtre 10 microns 56b), encadré par deux manomètres 57a et 57b, un filtre UV 58 (dont la fonction de stériliser l'eau pour éviter la diffusion de bactéries), une électrovanne de commande d'eau 59, un pressostat 60, un débistat 61 et un clapet anti-retour taré 62 agencé pour empêcher le fluide transporté de s'orienter vers l'arrivée d'eau 28.

En aval des clapets anti-retour 53 et 62, la conduite 25 d'alimentation en air et la conduite 27 d'alimentation en eau rejoignent la conduite principale d'alimentation 23 (entre l'accumulateur 40 et le clapet anti-retour 41) par un tronçon commun de conduite 63.

La conduite de sortie 29 s'étend depuis la conduite retour 24 (entre le raccord mâle/femelle 42 et l'électrovanne 43), jusqu'à la sortie d'évacuation 30, et elle comprend, dans le sens amont/aval du fluide transporté : une électrovanne de commande de sortie de fluide 64 et une vanne d'isolement 65.

Sur le circuit fluidique de la figure 5 on remarque encore :
- la présence d'un capteur capacitif 66 agencé pour détecter la présence/absence de liquide de traitement L dans le réservoir 22 ;
- une conduite 67, s'étendant entre la conduite d'alimentation principale 23, en aval de la pompe d'alimentation 35, et la conduite retour 24, juste en amont du réservoir 22, comprenant un limiteur de pression 68, adaptée pour maintenir une pression de service (par exemple de l'ordre de 100 bar) nécessaire à la production d'une bonne qualité de brouillard, et se prémunir de tout risque de surpression ; et
- une conduite 69, s'étendant entre la conduite retour 24, juste en amont du réservoir 22 et la conduite d'alimentation principale 23, juste en aval de la pompe de gavage 31, comprenant un limiteur de pression 70, adaptée pour s'assurer d'une bonne pression de service de la pompe de gavage 31 et se prémunir de tout risque de surpression (tarage à 2.5 bar).

Les différents composants du circuit fluidique de la figure 5 sont intégrés dans une armoire 71 (dite armoire hydraulique 71) agencée à proximité du caisson 2 (figures 1 et 2). Cette armoire hydraulique 71 est reliée au caisson 2 par la conduite d'alimentation principale 23 (au niveau de l'entrée 8 de la conduite interne 7) et par la conduite retour 24 (au niveau de la sortie 9 de la conduite interne 7) ; et elle est connectée à l'arrivée d'air 26 (par exemple une arrivée d'air sous une pression de 2 bar), à l'arrivée d'eau 28 (par exemple une arrivée d'eau à la pression de la ville, de l'ordre de 3 bar), et à la sortie d'évacuation 30 consistant par exemple en une évacuation vers l'égout ou une cuve de récupération en vue d'un traitement.

Le fonctionnement de l'installation de désinfection 1 est géré par un automate programmable (non représenté) intégré à l'armoire hydraulique 71 et muni d'une interface tactile.
Cet automate programmable gère la commande de la pompe de gavage 31, de la pompe haute pression 35, des électrovannes de commande d'eau 59, de commande d'air 50, de commande de retour 43 et de commande de sortie de fluide 64, des moyens de renouvellement/extraction d'air 14-20 et des gâches électriques qui équipent les portes d'entrée 4 et de sortie 5, cela en fonction d'un programme informatique adapté.

Bien entendu, tout autre schéma hydraulique permettant de remplir les fonctionnalités recherchées peut être envisagé.

### Procédé

Le produit liquide de traitement L utilisé dans le cadre de la mise en œuvre de l'installation de désinfection 1, pour la désinfection de chariots hospitaliers C, peut être du peroxyde d'hydrogène (H₂O₂), par exemple à une concentration de l'ordre de 6%. Bien entendu on peut utiliser toute autre concentration, ou tout autre produit adapté.

Le procédé de mise en œuvre de l'installation de désinfection 1 peut être le suivant :
Les chariots hospitaliers C à traiter sont introduits dans le volume de traitement 3 du caisson 2 par la porte d'entrée 4 ; et le caisson 2 est fermé de manière étanche (les portes 4 et 5 sont fermées manuellement et verrouillées, et les ouvertures d'entrée et de sortie d'air 14, 17 sont obturées).

### 1 - Désinfection :

On alimente ensuite la conduite interne 7 du caisson 2 par le liquide de traitement L, de manière adaptée pour diffuser ce liquide de traitement L dans le volume de traitement 3 par les buses de brumisation 10 afin de former un brouillard de traitement.

Pour cela, les électrovannes de commande d'eau 59, d'air 50 et de sortie 64 sont fermées ;

La pompe de gavage 31 et la pompe haute pression 35 sont démarrées, l'électrovanne 43 est ouverte et une temporisation de remplissage est activée, pour réaliser le remplissage de la conduite interne 7 par le liquide L.

Lorsque la temporisation de remplissage est atteinte, la conduite interne 7 est remplie par le liquide L et la brumisation de ce liquide L est réalisée dans le volume de traitement 3 pendant une durée déterminée (de l'ordre de quelques dizaines de secondes ou de quelques minutes) pour obtenir la saturation du volume de traitement 3 par le liquide de traitement L.

On laisse les pompes 31 et 35 en marche, on ferme l'électrovanne 43 et on active une temporisation de brumisation. Une fois cette temporisation de brumisation atteinte on arrête les pompes haute pression 35 et de gavage 31 et on conserve le brouillard de traitement en contact avec les chariots C pendant un temps de contact adapté pour assurer leur désinfection. Ce temps de contact peut aller de quelques minutes à quelques dizaines de minutes ; au besoin, une ou plusieurs séquences de brumisation complémentaires sont mises en œuvres pour conserver la saturation en produit de traitement L.

### 2 - Enlèvement ou vidage du liquide de traitement de la conduite interne :

Une fois le traitement de désinfection achevé, on alimente la conduite interne 7 par de l'air comprimé, de manière adaptée pour enlever le liquide de traitement L de cette conduite interne 7 et éventuellement des buses de brumisation 10.

Pour cela l'électrovanne d'air comprimé 50 et l'électrovanne de commande retour 43 sont ouvertes, et une temporisation de vidage est activée. Le reliquat de produit est évacué de la conduite interne 7 et est orienté vers le réservoir 22.

Une fois la temporisation de vidage atteinte l'opération de vidage est stoppée et les électrovannes d'air comprimé 50 et de commande retour 43 sont fermées.

### 3 - Rinçage de la conduite interne :

On alimente ensuite la conduite interne 7 par de l'eau, de manière adaptée pour assurer son rinçage, avec éventuellement le rinçage des buses de brumisation 10. Pour cela on commande en ouverture l'électrovanne de commande d'eau 59 et l'électrovanne de sortie de fluide 64 (les électrovannes de commande d'air 50 et de commande retour 43 étant fermées), et on déclenche une temporisation de rinçage. L'eau de rinçage est alors refoulée à l'égout ou dans un contenant adapté.

Une fois la temporisation de rinçage atteinte, le rinçage est terminé et on ferme l'électrovanne de commande d'eau 59.

Une opération de séchage de la conduite interne 7 est alors mise en œuvre par de l'air comprimé et refoulement à l'égout (ou dans le contenant adapté).

On laisse alors ouverte l'électrovanne de sortie de fluide 64 et on ouvre l'électrovanne de commande d'air 50 en activant une temporisation de séchage. Une fois la temporisation de séchage atteinte, le séchage est terminé et on ferme l'électrovanne de commande d'air 50, ainsi que l'électrovanne de sortie de fluide 64.

### 4 - Extraction de l'air vicié du volume de traitement :

L'air vicié est extrait du volume de traitement 3 par la commande de l'ouverture des moyens d'obturation amovibles 15 et 18 des ouvertures d'entrée 14 et de sortie 17 du caisson 2 et la mise en marche des moyens d'extraction 20.

En fonction du débit des moyens d'extraction 20 et du volume du caisson 2, cette opération peut durer de quelques minutes à quelques dizaines de minutes.

L'ai neuf apporté dans le volume de traitement 3 par la canalisation d'alimentation en air 16 permet de sécher les parois des chariots C et les parois internes du caisson 2.

Cette opération d'extraction peut débuter au cours de l'opération de vidage de la conduite interne 7 (opération 2 ci-dessus), par exemple dès l'initiation de cette opération de vidage ; et elle perdure avantageusement jusqu'à la fin du cycle, c'est-à-dire jusqu'à la fin de l'opération de séchage de la conduite interne 7 (ou éventuellement encore après la fin de ce séchage).

Une fois l'air vicié extrait du volume de traitement 3 les systèmes de verrouillage des portes 4 et 5 sont inactivés et les chariots hospitaliers C peuvent être enlevés du caisson 2 par la porte de sortie 5.

Les moyens de commande peuvent être adaptés pour commander également un éclairage interne du caisson 2 à des moments prédéfinis.

## Revendications

1. Installation pour le traitement de désinfection de chariots hospitaliers (C), en particulier des chariots (C) de transport de repas ou de linge,
laquelle installation (1) comprend :
- un caisson (2) délimitant un volume de traitement (3) adapté pour la réception d'une pluralité desdits chariots (C) et muni d'au moins une porte (4, 5) pour permettre l'entrée et la sortie desdits chariots (C), et
- des moyens de désinfection (6) comprenant au moins une conduite interne (7) munie d'une entrée (8) dans ledit caisson (2) et d'une sortie (9) dudit caisson (2), laquelle conduite interne (7) est munie de moyens de diffusion, disposés à l'intérieur dudit caisson (2), pour diffuser dans ledit volume de traitement (3) un produit liquide de traitement (L) contenant un agent de traitement hydrosoluble adapté au traitement de désinfection desdits chariots (C),
lesquels moyens de diffusion comprennent au moins une buse de brumisation (10),
lesquels moyens de désinfection (6) comprennent des moyens (11) permettant d'alimenter ladite entrée (8) de conduite interne (7) avec ledit produit liquide de traitement (L),
laquelle installation comprend encore une canalisation (16) adaptée pour alimenter en air ledit volume de traitement (3) et une canalisation (19) équipée de moyens d'extraction (20) adaptés pour extraire l'air vicié dudit volume de traitement (3),
et lequel caisson (2) comprend au moins une ouverture d'entrée (14), munie de moyens d'obturation amovibles (15), associée à ladite canalisation (16) adaptée pour alimenter en air ledit volume de traitement (3), et au moins une ouverture de sortie (17), munie de moyens d'obturation amovibles (18), associée à ladite canalisation (19) équipée desdits moyens d'extraction (20) adaptés pour extraire l'air vicié dudit volume de traitement (3),
**caractérisée en ce que** lesdits moyens de désinfection (6) comprennent encore :
- des moyens (13) permettant d'alimenter ladite entrée (8) de conduite interne (7) avec de l'air, et
- des moyens (12) permettant d'alimenter ladite entrée (8) de conduite interne (7) avec de l'eau.

2. Installation selon la revendication 1, **caractérisée en ce que** ledit caisson (2) comporte un module d'entrée (2a) muni d'une porte d'entrée (4), et un module de sortie (2b) muni d'une porte de sortie (5), entre lesquels sont ménagés une pluralité de modules intermédiaires (2c) identiques, dont le nombre est fonction du volume de traitement (3) recherché dudit caisson (2), ledit module d'entrée (2a) et ledit module de sortie (2b) étant assemblés de manière étanche avec ledit module intermédiaire (2c) adjacent, et lesdits modules intermédiaires (2c) étant assemblés entre eux de manière étanche.

3. Installation selon la revendication 2, **caractérisée en ce qu'**elle comprend au moins une buse de brumisation (10) ménagée sur ladite conduite interne (7) au niveau dudit module d'entrée (2a), au dessus du niveau de ladite porte d'entrée (4), et au moins une buse de brumisation (10) ménagée sur ladite conduite interne (7) au niveau dudit module de sortie (2b), au dessus du niveau de ladite porte de sortie (5).

4. Installation selon l'une quelconque des revendications 1 à 3, **caractérisée en ce qu'**elle comprend des moyens de commande permettant de commander :
- l'alimentation de ladite entrée (8) de conduite interne (7), soit par le produit liquide de traitement (L), soit par de l'eau, soit par de l'air, et/ou
- l'ouverture desdits moyens d'obturation amovibles (15, 18) des ouvertures d'entrée (14) et de sortie (17) du caisson (2) et la mise en marche desdits moyens d'extraction (20), pour extraire l'air vicié dudit volume de traitement (3).

5. Installation selon la revendication 4, **caractérisée en ce qu'**elle comporte un circuit fluidique comprenant :
- un réservoir (22) de produit de liquide de traitement (L) situé à l'extérieur dudit caisson (2),
- une conduite principale d'alimentation (23) s'étendant depuis ledit réservoir (22) de produit liquide de traitement (L) jusqu'à ladite entrée (8) de conduite interne (7),
- une conduite retour (24) s'étendant depuis ladite sortie (9) de conduite interne (7) jusqu'audit réservoir (22) de produit liquide de traitement (L),
- une conduite d'alimentation en air (25) s'étendant depuis une arrivée d'air (26) jusqu'à ladite conduite principale d'alimentation (23),
- une conduite d'alimentation en eau (27) s'étendant depuis une arrivée d'eau (28) jusqu'à ladite conduite principale d'alimentation (23), et
- une conduite de sortie (29) s'étendant depuis ladite sortie (9) de conduite interne (7) jusqu'à une sortie d'évacuation (30),
différents clapets anti-retour (39, 44, 53, 62) étant agencés sur ledit circuit fluidique pour assurer le transport du produit liquide de traitement (L), de l'eau et de l'air, uniquement dans une direction en amont et en aval de ladite conduite interne (7),
et différentes vannes (43, 50, 59, 64) étant agencées sur ledit circuit fluidique, dont la position d'ouverture/fermeture est gérée par lesdits moyens de commande, adaptées pour autoriser :
- l'alimentation de ladite entrée (8) de conduite interne (7), soit par le produit liquide de traitement (L), soit par de l'eau, soit par de l'air,
- l'évacuation de l'eau vers ladite sortie d'évacuation (30),
- le retour dudit produit liquide de traitement (L) et de l'air dans ledit réservoir (22).

6. Installation selon la revendication 5, **caractérisée en ce qu'**elle comporte une pompe d'alimentation (35) placée sur ladite conduite principale d'alimentation (23) pour assurer l'alimentation de ladite entrée (8) de conduite interne (7) par le produit liquide de traitement (L),
et **en ce que** ladite conduite d'alimentation en air (25) et ladite conduite d'alimentation en eau (27) viennent se raccorder à ladite conduite principale d'alimentation (23) entre ladite pompe d'alimentation (35) et ladite entrée (8) de conduite interne (7).

7. Installation selon la revendication 6, **caractérisée en ce qu'**elle comporte une pompe de gavage (31) placée sur ladite conduite principale d'alimentation (23) entre ledit réservoir (22) de produit de liquide de traitement et ladite pompe d'alimentation (35).

8. Installation selon l'une quelconque des revendications 5 à 7, **caractérisée en ce qu'**elle comporte des moyens (45) de détection de la présence de liquide dans ladite conduite retour (24), lesdits moyens de commande étant adaptés pour commander la diffusion du produit liquide de traitement (L) par la ou les buses de brumisation (10) uniquement en cas de détection de liquide dans ladite conduite retour (24).

9. Procédé de désinfection de chariots hospitaliers (C) au moyen d'une installation (1) selon l'une quelconque des revendications 1 à 8, **caractérisé en ce qu'**il comprend les étapes consistant à :
a/ introduire les chariots hospitaliers (C) dans le volume de traitement (3) du caisson (2),
b/ fermer ledit caisson (2) de manière étanche,
c/ alimenter ladite conduite interne (7) par ledit produit liquide de traitement (L), de manière adaptée pour diffuser ledit produit liquide de traitement (L) dans ledit volume de traitement (3) par la ou les buses de brumisation (10) et former ainsi un brouillard de traitement,
d/ conserver le brouillard de traitement en contact avec lesdits chariots hospitaliers (C) pendant un temps de contact adapté pour assurer leur désinfection,
e/ alimenter ladite conduite interne (7) par de l'air, de manière adaptée pour enlever ledit produit liquide de traitement (L) de ladite conduite interne (7) et éventuellement desdites buses de brumisation (10),
f/ alimenter ladite conduite interne (7) par de l'eau, de manière adaptée pour assurer son rinçage et éventuellement le rinçage desdites buses de brumisation (10), et
simultanément aux étapes e/ et/ou f/, ou après l'étape f/ :
g/ commander l'ouverture desdits moyens d'obturation amovibles (15, 18) des ouvertures d'entrée (14) et de sortie (17) du caisson (2) et la mise en marche desdits moyens d'extraction (20), pour extraire l'air vicié dudit volume de traitement (3), et
h/ sortir lesdits chariots hospitaliers (C) dudit caisson (2).

10. Procédé de désinfection de chariots hospitaliers (C) selon la revendication 9, **caractérisé en ce qu'**il comprend une étape de remplissage complet de la conduite interne (7) par ledit produit liquide de traitement (L), avant d'assurer la diffusion dudit produit liquide de traitement (L) par lesdites buses de brumisation (10).

## Patentansprüche

1. Anlage zur Desinfektionsbehandlung von Krankenhauswagen (C), insbesondere von Wagen (C) zum Transport von Essen oder Wäsche,
wobei die Anlage (1)
- einen Kasten (2), der ein Behandlungsvolumen (3) umgrenzt, das zur Aufnahme einer Anzahl Wagen (C) ausgelegt ist und mit wenigstens einer Tür (4, 5) versehen ist, um das Hineinfahren und Hinausfahren der Wagen (C) zu ermöglichen, und
- Desinfektionsmittel (6), die wenigstens eine innere Leitung (7) aufweisen, die mit einem Einlaß (8) in den Kasten (2) und einem Auslaß (9) aus dem Kasten (2) versehen ist, wobei die innere Leitung (7) mit im Inneren des Kastens (2) angeordneten Verteilungsmitteln versehen ist, um in dem Behandlungsvolumen (3) ein flüssiges Behandlungsmittel (L) zu verteilen, das einen wasserlöslichen Behandlungsstoff enthält, der an die Desinfektionsbehandlung der Wagen (C) angepaßt ist,
aufweist,
wobei die Verteilungsmittel wenigstens eine Zerstäuberdüse (10) aufweisen, wobei die Desinfektionsmittel (6) Mittel (11) aufweisen, die ermöglichen, den Eingang (8) der inneren Leitung (7) mit dem flüssigen Behandlungsmittel (L) zu versorgen,
wobei die Anlage außerdem eine Kanalisation (16), die zum Versorgen des Behandlungsvolumens (3) mit Luft ausgelegt ist, und eine Kanalisation (19), die mit Abzugsmitteln (20) ausgestattet ist, die zum Abziehen der verbrauchten Luft aus dem Behandlungsvolumen (3) ausgelegt sind, aufweist,
und wobei der Kasten (2) wenigstens eine Zugangsöffnung (14), die mit abnehmbaren Verschlußmitteln (15) versehen ist und der Kanalisation (16), die zum Versorgen des Behandlungsvolumens (3) mit Luft ausgelegt ist, zugeordnet ist, und wenigstens eine Ausgangsöffnung (17), die mit abnehmbaren Verschlußmitteln (18) versehen ist und der mit den Abzugsmitteln (20), die zum Abziehen der verbrauchten Luft aus dem Behandlungsvolumen (3) ausgelegt sind, versehenen Kanalisation (19) zugeordnet ist, aufweist,
**dadurch gekennzeichnet, daß** die Desinfektionsmittel (6) außerdem
- Mittel (13), die ein Versorgen des Eingangs (8) der inneren Leitung (7) mit Luft ermöglichen, und
- Mittel (12), die ein Versorgen des Eingangs (8) der inneren Leitung (7) mit Wasser ermöglichen,
aufweisen.

2. Anlage gemäß Anspruch 1, **dadurch gekennzeichnet, daß** der Kasten (2) ein mit einer Eingangstür (4) versehenes Eingangsmodul (2a) und ein mit einer Ausgangstür (5) versehenes Ausgangsmodul (2b) aufweist, zwischen denen eine Anzahl identischer Zwischenmodule (2c) eingerichtet sind, deren Zahl vom zu erzielenden Behandlungsvolumen (3) des Kastens (2) abhängt, wobei das Eingangsmodul (2a) und das Ausgangsmodul (2c) mit dem angrenzenden Zwischenmodul (2c) dicht verbunden sind und wobei die Zwischenmodule (2c) untereinander dicht verbunden sind.

3. Anlage gemäß Anspruch 2, **dadurch gekennzeichnet, daß** sie wenigstens eine Zerstäuberdüse (10), die auf der inneren Leitung (7) im Bereich des Eingangsmoduls (2a) oberhalb der Ebene der Eingangstür (4) angeordnet ist, und wenigstens eine Zerstäuberdüse (10), die auf der inneren Leitung (7) im Bereich des Ausgangsmoduls (2b) oberhalb der Ebene der Ausgangstür (5) angeordnet ist, aufweist.

4. Anlage gemäß einem der Ansprüche 1 bis 3, **dadurch gekennzeichnet, daß** sie Steuerungsmittel aufweist, die ermöglichen,
- die Versorgung des Eingangs (8) der inneren Leitung (7) entweder mit dem flüssigen Behandlungsmittel (L) oder mit Wasser oder mit Luft und/oder
- die Öffnung der abnehmbaren Verschlußmittel (15, 18) der Eingangs- (14) und Ausgangsöffnungen (17) des Kastens (2) und das Ingangsetzen der Abzugsmittel (20), um die verbrauchte Luft aus dem Behandlungsvolumen (3) abzuziehen,
zu steuern.

5. Anlage gemäß Anspruch 4, **dadurch gekennzeichnet, daß** sie einen Flüssigkeitskreislauf aufweist, der
- einen außerhalb des Kastens (2) gelegenen Tank (22) für ein flüssiges Behandlungsmittel (L),
- eine Hauptversorgungsleitung (23), die sich von dem Tank (22) für ein flüssiges Behandlungsmittel (L) bis zum Eingang (8) der inneren Leitung (7) erstreckt,
- eine Rückleitung (24), die sich vom Ausgang (9) der inneren Leitung (7) bis zum Tank (22) für ein flüssiges Behandlungsmittel (L) erstreckt,
- eine Luftversorgungsleitung (25), die sich von einem Lufteinlaß (26) bis zur Hauptversorgungsleitung (23) erstreckt,
- eine Wasserversorgungsleitung (27), die sich von einem Wasserzufluß (28) bis zur Hauptversorgungsleitung (23) erstreckt, und
- eine Ausgangsleitung (29), die sich vom Ausgang (9) der inneren Leitung (7) bis zu einem Ablaßausgang (30) erstreckt,
aufweist,
wobei verschiedene Rückschlagventile (39, 44, 53, 62) im Flüssigkeitskreislauf angeordnet sind, um den Transport des flüssigen Behandlungsmittels (L), des Wassers und der Luft stromaufwärts und stromabwärts der inneren Leitung (7) in nur einer Richtung sicherzustellen,
und wobei verschiedene Ventile (43, 50, 59, 64) im Flüssigkeitskreislauf angeordnet sind, deren Öffnungs-/Schließstellung durch die Steuerungsmittel gesteuert wird und die dazu ausgelegt sind,
- die Versorgung des Eingangs (8) der inneren Leitung (7) entweder mit dem flüssigen Behandlungsmittel (L) oder Wasser oder Luft,
- die Entsorgung des Wassers zum Ablaßausgang (30) hin,
- die Rückführung des flüssigen Behandlungsmittels (L) und der Luft in den Tank (22)
zuzulassen.

6. Anlage gemäß Anspruch 5, **dadurch gekennzeichnet, daß** sie eine auf der Hauptversorgungsleitung (23) angeordnete Versorgungspumpe (35) aufweist, um die Versorgung des Eingangs (8) der inneren Leitung (7) mit dem flüssigen Behandlungsmittel (L) sicherzustellen,
und daß die Luftversorgungsleitung (25) und die Wasserversorgungsleitung (27) zwischen der Versorgungspumpe (35) und dem Eingang (8) der inneren Leitung (7) an die Hauptversorgungsleitung (23) angeschlossen sind.

7. Anlage gemäß Anspruch 6, **dadurch gekennzeichnet, daß** sie eine auf der Hauptversorgungsleitung (23) zwischen dem Tank (22) für ein flüssiges Behandlungsmittel und der Versorgungspumpe (35) angeordnete Förderpumpe (31) aufweist.

8. Anlage gemäß einem der Ansprüche 5 bis 7, **dadurch gekennzeichnet, daß** sie Mittel (45) zum Erfassen des Vorhandenseins von Flüssigkeit in der Rückleitung (24) aufweist, wobei die Steuerungsmittel dazu ausgelegt sind, die Verteilung des flüssigen Behandlungsmittels (L) durch die Zerstäuberdüse oder -düsen (10) nur im Fall des Erfassens von Flüssigkeit in der Rückleitung (24) zu steuern.

9. Verfahren zum Desinfizieren von Krankenhauswagen (C) mittels einer Anlage (1) gemäß einem der Ansprüche 1 bis 8, **dadurch gekennzeichnet, daß** es die Schritte aufweist, die darin bestehen:
a) die Krankenhauswagen (C) in das Behandlungsvolumen (3) des Kastens (2) hineinzubringen,
b) den Kasten (2) dicht zu schließen,
c) die innere Leitung (7) in geeigneter Weise mit dem flüssigen Behandlungsmittel (L) zu versorgen, um das flüssige Behandlungsmittel (L) im Behandlungsvolumen (3) durch die Zerstäuberdüse oder -düsen (10) zu verteilen und so einen Behandlungsnebel zu erzeugen,
d) den Behandlungsnebel während einer angepaßten Kontaktdauer mit den Krankenhauswagen (C) in Kontakt zu halten, um deren Desinfizierung sicherzustellen,
e) die innere Leitung (7) in angepaßter Weise mit Luft zu versorgen, um das flüssige Behandlungsmittel (L) aus der inneren Leitung und eventuell aus der Zerstäuberdüse bzw. den Zerstäuberdüsen (10) zu entfernen,
f) die innere Leitung (7) in angepaßter Weise mit Wasser zu versorgen, um deren Spülung und eventuell die Spülung der Zerstäuberdüse oder -düsen (10) sicherzustellen, und
gleichzeitig mit den Schritten e) und/oder f) oder nach f)
g) die Öffnung der abnehmbaren Verschlußmittel (15, 18) der Eingangs- (14) und Ausgangsöffnungen (17) des Kastens (2) und das Ingangsetzen der Abzugsmittel (20), um die verbrauchte Luft aus dem Behandlungsvolumen (3) abzuziehen, zu steuern und
h) die Krankenhauswagen (C) aus dem Kasten (2) herauszubringen,

10. Verfahren zum Desinfizieren von Krankenhauswagen (C) gemäß Anspruch 9, **dadurch gekennzeichnet, daß** es einen Schritt des vollständigen Befüllens der inneren Leitung (7) mit dem flüssigen Behandlungsmittel (L) aufweist, bevor die Verteilung des flüssigen Behandlungsmittels (L) durch die Zerstäuberdüsen (10) sichergestellt wird.

## Claims

1. A facility for disinfection treatment of hospital carts (C), in particular meal or linen transport carts (C),
wherein said facility (1) comprises:
- a casing (2) delimiting a treatment volume (3) for receiving a plurality of said carts (C) and provided with at least one door (4, 5) for entry and exit of said carts (C), and
- disinfection means (6) comprising at least one internal pipe (7) provided with an inlet (8) to said casing (2) and an outlet (9) from said casing (2), wherein said internal pipe (7) is provided with diffusion means, arranged inside said casing (2), to diffuse into said treatment volume (3) a liquid treatment product (L) containing a water-soluble treatment agent suitable for disinfection treatment of said carts (C),
wherein said diffusion means comprise at least one misting nozzle (10),
wherein said disinfection means (6) comprise means (11) for supplying said internal pipe (7) inlet (8) with said liquid treatment product (L),
wherein said facility also comprises a pipe (16) for supplying said treatment volume (3) with air and a pipe (19) fitted with extraction means (20) for extracting the stale air from said treatment volume (3),
and wherein said casing (2) comprises at least one inlet (14), provided with removable plugging means (15), associated with said pipe (16) for supplying said treatment volume (3) with air, and at least one outlet (17), provided with removable plugging means (18), associated with said pipe (19) fitted with said extraction means (20) for extracting the stale air from said treatment volume (3),
**characterized in that** said disinfection means (6) also comprise:
- means (13) for supplying said internal pipe (7) inlet (8) with air, and
- means (12) for supplying said internal pipe (7) inlet (8) with water.

2. The facility according to claim 1, **characterized in that** said casing (2) includes an entrance module (2a) provided with an entrance door (4), and an exit module (2b) provided with an exit door (5), between which are provided a plurality of identical intermediate modules (2c), whose number is function of the desired treatment volume (3) for said casing (2), said entrance module (2a) and said exit module (2b) being tightly assembled to said adjacent intermediate module (2c), and said intermediate modules (2c) being tightly assembled to each other.

3. The facility according to claim 2, **characterized in that** it comprises at least one misting nozzle (10) arranged on said internal pipe (7) at said entrance module (2a), above the level of said entrance door (4), and at least one misting nozzle (10) arranged on said internal pipe (7) at said exit module (2b), above the level of said exit door (5).

4. The facility according to any one of claims 1 to 3, **characterized in that** it comprises control means for controlling:
- the supply of said inner pipe (7) inlet (8) either with the liquid treatment product (L), or with water, or with air, and/or
- the opening of said removable plugging means (15, 18) of the inlet (14) and outlet (17) of the casing (2) and the start-up of said extraction means (20), for extracting the stale air from said treatment volume (3).

5. The facility according to claim 4, **characterized in that** it includes a fluidic circuit comprising:
- a tank (22) for liquid treatment product (L) located outside said casing (2),
- a main supply pipe (23) extending from said tank (22) for liquid treatment product (L) to said internal pipe (7) inlet (8),
- a return pipe (24) extending from said internal pipe (7) outlet (9) to said tank (22) for liquid treatment product (L),
- an air supply pipe (25) extending from an air inlet (26) to said main supply pipe (23),
- a water supply pipe (27) extending from a water inlet (28) to said main supply pipe (23), and
- an exit pipe (29) extending from said internal pipe (7) outlet (9) to an evacuation outlet (30),
different check valves (39, 44, 53, 62) being arranged on said fluidic circuit for the transport of the liquid treatment product (L), the water and the air, only in one direction upstream and downstream of said internal pipe (7),
and different valves (43, 50, 59, 64) being arranged on said fluid circuit, whose opening/closing position is managed by said control means, for allowing:
- the supply of said internal pipe (7) inlet (8), either with the liquid treatment product (L), or with water, or with air,
- the evacuation of the water towards said evacuation outlet (30),
- the return of said liquid treatment product (L) and air into said tank (22).

6. The facility according to claim 5, **characterized in that** it includes a supply pump (35) placed on said main supply pipe (23) for supplying said internal pipe (7) inlet (8) with the liquid treatment product (L),
and **in that** said air supply pipe (25) and said water supply pipe (27) are connected to said main supply pipe (23) between said supply pump (35) and said internal pipe (7) inlet (8).

7. The facility according to claim 6, **characterized in that** it includes a boost pump (31) placed on said main supply pipe (23), between said tank (22) for liquid treatment product (L) and said supply pump (35).

8. The facility according to any one of claims 5 to 7, **characterized in that** it includes means (45) for detecting the presence of liquid in said return pipe (24), said control means being adapted to control the diffusion of the liquid treatment product (L) through the misting nozzle(s) (10) only in case of detection of liquid in said return pipe (24).

9. A method for disinfection of hospital carts (C) by means of a facility (1) according to any one of claims 1 to 8, **characterized in that** it comprises the following steps:
a) introducing the hospital carts (C) into the treatment volume (3) of the casing (2),
b) tightly closing said casing (2),
c) supplying said internal pipe (7) with said liquid treatment product (L), in such a way as to diffuse said liquid treatment product (L) into said treatment volume (3) through the misting nozzle(s) (10) and to hence form a treatment mist,
d) keeping the treatment mist in contact with said hospital carts (C) for a contact time suitable for providing the disinfection thereof,
e) supplying said internal pipe (7) with air, in such a way as to remove said liquid treatment product (L) from said internal pipe (7) and potentially said misting nozzles (10),
f) supplying said internal pipe (7) with water, in such a way as to rinse it and potentially rinse said misting nozzles (10), and
simultaneously to steps e) and/or f), of after step f):
g) controlling the opening of said removable plugging means (15, 18) of the inlet (14) and outlet (17) of the casing (2) and the start-up of said extraction means (20) for extracting the stale air from said treatment volume (3), and
h) exiting said hospital carts (C) from said casing (2).

10. The method for disinfection of hospital carts (C) according to claim 9, **characterized in that** it comprises a step of entirely filling the internal pipe (7) with said liquid treatment product (L), before diffusing said liquid treatment product (L) through said misting nozzles (10).
